# EUROPÄISCHE PATENTSCHRIFT

(11) **EP 1 025 428 B1**
(45) Veröffentlichungstag und Bekanntmachung des Hinweises auf die Patenterteilung: **11.09.2002**
(21) Anmeldenummer: 97953804.8
(22) Anmeldetag: 12.12.1997
(51) Int. Cl.: G01N 1/28

(54) **VERFAHREN UND VORRICHTUNG ZUR MIKROPARTIKELPOSITIONIERUNG IN FELDKÄFIGEN**
METHOD AND DEVICE FOR MICRO PARTICLE POSITIONING IN FIELD CAGES
PROCEDE ET DISPOSITIF POUR POSITIONNER DES MICROPARTICULES DANS DES CAGES DE CHAMPS

(30) Priorität: 20.12.1996 DE 19653661
(43) Veröffentlichungstag der Anmeldung: 09.08.2000
(73) Patentinhaber: Evotec OAI AG, 22525 Hamburg (DE)
(72) Erfinder: FUHR, Günter, D-13187 Berlin (DE)
(74) Vertreter: Hertz, Oliver, Dr.
(86) Internationale Anmeldenummer: EP9707001
(87) Internationale Veröffentlichungsnummer: WO98028604

(56) Entgegenhaltungen:
- EP-A- 0 453 780
- DE-A- 4 400 955
- DE-A- 19 500 660

## Beschreibung

Die Erfindung betrifft ein Verfahren zur Steuerung der Position bzw. Bewegung von Objekten in Feldkäfigen und eine Vorrichtung zur Durchführung des Verfahrens.

Bei zahlreichen biologischen, medizinischen, pharmakologischen oder physikalisch- bzw. chemisch-technischen Prozessen besteht ein Interesse daran, mikroskopisch kleine Teilchen, insbesondere biologische Zellen oder Bestandteile von diesen, Latexpartikeln oder andere Microbeads, möglichst positionsgenau zu haltern oder entlang einer bestimmten Bahn zu führen. Eine bekannte Technik zur Halterung biologischer Zellen besteht im Aufwachsen der Zellen auf einem festen Substrat, das dann mit der geforderten Genauigkeit z.B. in Bezug auf ein Meßmittel eingestellt werden kann. Der Nachteil dieser Technik besteht in der Bildung eines mechanischen Kontakts mit der Substratoberfläche und der generell schwierigen Trennung der Verbindung z.B. nach Durchführung einer Messung. Außerdem ist diese Technik auf die Halterung von Objekten beschränkt und nicht zur Führung entlang vorbestimmter Bahnen geeignet.

Es ist ferner bekannt, Objekte in freien Flüssigkeiten mit optischen Mitteln zu positionieren. Mit einem sogenannten Laserstrahl-Tweezer ist es möglich, Partikel in freien Lösungen in mikrometergenauen Positionen zu halten oder in vorbestimmter Weise zu verschieben (s. A. Ashkin et al. "Observation of a single-beam gradient force optical trap for dielectric particles" in: Optics Lett., 11, 288 (1986)). Dieses sogenannte optische Trapping ist nachteilig, da das Teilchen innerhalb des halternden Laserstrahls thermischen Stößen ausgesetzt ist und somit statistisch geringfügigen Positionsschwankungen unterliegt. Außerdem sind optische Messungen am gehalterten Teilchen wegen der Interferenz mit dem halternden Laserstrahl nur eingeschränkt möglich. Letztere Beschränkung wird bei der Manipulierung von Teilchen in elektrischen Mikrofeldkäfigen vermieden, in denen die Objekte über Polarisationskräfte gehaltert oder bewegt werden können (s. G. Fuhr et al., "Radio-frequency microtools for particle and live cell manipulation" in: Naturwissenschaften 81, 528 (1994), oder : DE-OS 19 500 660). Allerdings treten auch hier thermisch bedingte Positionsschwankungen der gehalterten oder bewegten Teilchen auf, wodurch hochauflösende oder hochempfindliche Messungen, wie z.B. Verfahren der Korrelationsspektroskopie (s. M. Eigen et al. "Sorting single molecules, application to diagnostics and evolutionary biotechnology" in Proc. nat. Acad. Sci. USA 91, 5740 (1994)), erschwert oder ganz verhindert werden.

Es ist bekannt, das zu halternde Teilchen in der gewünschten Position in Schwingung zu versetzen, um ortsaufgelöste Messungen unter verminderter Störung durch thermische Stöße zu ermöglichen. Für diesen Zweck wird das gehalterte Teilchen in der gewünschten Position mittels Schall (insbesondere Ultraschall) oder mittels periodischer Lösungsbewegungen in Schwingung versetzt, so daß eine bestimmte periodische Bewegung am Ort der gewünschten Position erfolgt. Das entsprechend modulierte Meßsignal besitzt ein verbessertes Signal-Rausch-Verhalten.

Diese Vibrationstechnik hat jedoch die folgenden Nachteile. Die Kräfte zur Ausbildung der Schwingung wirken indirekt über die Umgebungsflüssigkeit auf das gehalterte Teilchen. Damit wird nicht nur das Teilchen, sondern auch die Umgebungsflüssigkeit bewegt, wodurch die Reproduzierbarkeit der Teilchenpositionierung vermindert ist. Ferner können bei dieser indirekten Krafteinwirkung unerwünschte und schwer kontrollierbare Drehbewegungen auftreten. Bestimmte Anwendungen, bei denen eine Umgebungsflüssigkeitsbewegung unerwünscht sind, sind nicht realisierbar. Schließlich kann am gehalterten Teilchen mechanischer Streß auftreten, der insbesondere bei biologischen lebenden Objekten problematisch ist.

Mit den bekannten Techniken ist es nicht möglich, die Positionierung eines Teilchens entlang einer gewünschten Teilchenbahn zu beeinflussen. Es besteht jedoch auch ein Bedarf an über die o.a. Vibrationstechnik hinausgehenden Bewegungssteuerungen.

Aufgabe der Erfindung ist es daher, ein verbessertes Verfahren zur Positionierung von Objekten mit elektrischen Feldkäfigen und eine Vorrichtung zur Durchführung des Verfahrens bereitzustellen, mit denen die Einsatzmöglichkeiten der genannten Virbrationstechnik erweitert und neue Steuerungsmöglichkeiten geschaffen werden können.

Diese Aufgabe wird durch ein Verfahren mit den Merkmalen gemäß Patentanspruch 1 und eine Vorrichtung mit den Merkmalen gemäß Patentanspruch 9 gelöst. Vorteilhafte Formen der Erfindung ergeben sich aus den abhängigen Ansprüchen.

Der Erfindung liegt die Idee zugrunde, bei der Positionierung mit Schwingungsüberlagerung oder bei anderen Bewegungssteuerungen vom Einsatz der mittelbar über die Flüssigkeitsbewegung vermittelten Kräfte abzugehen und stattdessen das Objekt unter Wirkung von Kräften aperiodisch oder periodisch zu bewegen, die direkt auf das gehalterte Objekt, nicht jedoch auf die Umgebungsflüssigkeit (Suspension, Lösung) wirken. Hierzu werden elektrische Potentiale an Elektroden einer Mehrelektrodenanordnung zur Bildung eines offenen oder geschlossenen Feldkäfigs mit derartigen Steuerpotentialen überlagert, daß sich die Position des Feldminimums, in dem sich das jeweilige Objekt befindet, in Bezug auf die gewünschte Objektposition ändert. Die gewünschte Objektposition kann entweder ein bestimmter Ort innerhalb eines geschlossenen Feldkäfigs oder eine Bahn innerhalb eines offenen Feldkäfigs (Kanalstruktur oder dgl.) sein. Eine periodische Positionsänderung bedeutet, daß das gehalterte Teilchen periodisch auf einer Bahn um die gewünschte, vorbestimmte Objektposition geführt wird. Die Form dieser überlagerten Bewegungsbahn ist beliebig wählbar und kann eine oszillatorische Bewegung in Bezug auf eine, zwei oder drei Raumachsen, kreisförmige, elliptische, im wesentlichen rechteckige oder auch kompliziertere Bahnen umfassen. Das Zeitmuster der räumlich periodischen Bewegung kann selbst eine periodische Modulation aufweisen. Eine aperiodische Positionsänderung bedeutet, daß das gehalterte Teilchen auf einer bestimmten Bahn von einer vorbestimmten Objektposition weggeführt wird.

Falls die gewünschte Objektposition durch eine Bahn innerhalb eines offenen Feldkäfigs gebildet wird, so kann die Bewegung des Objekts entlang der gewünschten Bahn entweder unter geeignet gewählter Ansteuerung der Elektroden mit Grundpotentialen und/oder durch eine Flüssigkeitsströmung erfolgen.

Eine erfindungsgemäße Vorrichtung besitzt eine Elektrodenanordnung, die mit einer Kombination von HF-Generatoren und einem Schaltmittel ansteuerbar sind. Das Schaltmittel ist dazu eingerichtet, das Potential (sog. Grundpotential) einer oder mehrerer Elektroden mit einem Steuerpotential zu überlagern, so daß sich die Amplitude, die Frequenz und/oder die Phasenlage des sich ergebenden Gesamtpotentials verändert.

Mit der Erfindung können die folgenden Vorteile insbesondere bei der Positionierung von Objekten, die in Feldkäfigen mit Umhüllungsflüssigkeiten frei getragen werden, erzielt werden.

Die Positionierung bzw. Bewegung der Objekte erfolgt ohne mechanische Berührung. Die Objekte werden keinem störenden mechanischen Streß ausgesetzt, so daß insbesondere lebende biologische Zellen oder dgl. nicht geschädigt werden. Es gibt keinerlei Beschränkung hinsichtlich der weiteren Manipulierung oder Vermessung der gehalterten Objekte. Es sind insbesondere übliche optische Meßmethoden unter Vermeidung von Interferenzproblemen einsetzbar. Die Bewegung der Objekte um den gewünschten Ort bzw. die gewünschte Bahn kann in reproduzierbarer Weise, abweichend von harmonischen Oszillationsabläufen komplex gestaltet sein. Die überlagerte Bewegung der Objekte kann in vorbestimmter Weise gesteuert bzw. programmiert werden. Es besteht ein eindeutiger Zusammenhang zwischen der Gestaltung der Steuerpotentiale und den hervorgerufenen Objektbewegungen, so daß keine Rückkopplungskontrolle oder Beobachtung des bewegten Objekts zur Regelung erforderlich sind. Schließlich ist die Erfindung mit beliebigen Elektrodenanordnungen zur Bildung von Feldkäfigen unabhängig von der konkreten Elektrodenform oder -anordnung anwendbar.

Erfindungsgemäße Verfahren und Vorrichtungen können vorzugsweise für die Korrelationspektroskopie, insbesondere zum Nachweis fluoreszierender Moleküle an der Oberfläche von Submikrometer- oder Mikrometer-Teilchen und/oder -Zellen, oder für pharmakologische, medizinisch-diagnostische und/oder evolutions-biotechnologische Anwendungen benutzt werden. Insbesondere können als Nachweisverfahren die Methode der Fluoreszenz-Korrelationsspektroskopie (WO 94/16313) sowie andere, insbesondere konfokale Fluoreszenztechniken, wie in der Offenlegungsschrift WO 96/13744 und der europäischen Patentanmeldung 96116373.0 vorgeschlagen, eingesetzt werden. Die letztgenannte Anmeldung schlägt ein Verfahren zur Analyse von Proben durch wiederholte Messung der Anzahl von Photonen pro definiertem Zeitintervall von elektromagnetischer Strahlung, insbesondere Licht, vor, welche von den Partikeln in der Probe emittiert, gestreut und/oder reflektiert wird, und Bestimmung der Verteilung der Anzahl von Photonen in den jeweiligen Zeitintervallen, wobei die Verteilung der molekularen Helligkeiten der Partikel aus der Verteilung der Anzahl von Photonen bestimmt wird.

Ausführungsformen der Erfindung werden im folgenden unter Bezug auf die beigefügten Zeichnungen erläutert. Es zeigen:
- Fig. 1: eine schematische Perspektivansicht eines drei-dimensionalen Feldkäfigs mit einer Blockdarstellung einer Ansteuerschaltung gemäß einer Ausführungsform der Erfindung;
- Fig. 2: eine schematische Perspektivansicht eines offenen Feldkäfigs zur Illustration von erfindungsgemäß modifizierten Teilchenbahnen; und
- Fig. 3: eine schematische Perspektivansicht einer Elektrodenanordnung für zwei benachbarte, offene Feldkäfige.

Die im folgenden unter Bezug auf einzelne Ausführungsbeispiele erläuterten Einzelmerkmale der erfindungsgemäßen Verfahrensweise bzw. der Steuerschaltung sind nicht auf die jeweiligen Ausführungsbeispiele beschränkt. Dem Fachmann ist es vielmehr möglich, anwendungsabhängig Modifikationen der dargestellten Beispiele insbesondere hinsichtlich der Amplituden, Frequenzen und Phasenlage der Grund- und Steuerpotentiale der Elektroden auszuwählen.

Zur Realisierung der erfindungsgemäßen Verfahrensweise werden Elektrodenanordnungen verwendet, wie sie zur Ausbildung von Feldkäfigen insbesondere aus der Mikrosystemtechnik bekannt sind und vorzugsweise mit Methoden der Halbleitertechnologie hergestellt werden. Im Falle zweidimensionaler Elektrodenanordnungen sind die Elektroden auf einer Chipoberfläche mit charakteristischen Größen im nm- bis µm-Bereich gebildet. Dreidimensionale Elektrodenanordnungen können aus zwei planaren, zweidimensionalen Anordnungen bestehen, die unter Verwendung von strukturierten Abstandshaltermitteln zur Bildung von Kanälen, Reservoirs oder anderen durchströmbaren Räumen zusammengefügt sind.

Mikroelektroden (Querschnittsdimension 50 nm bis einige Mikrometer, Länge anwendungsabhängig) werden so mit elektrischen Grundpotentialen beaufschlagt, daß sich im dem zwischen den Mikroelektroden gebildeten Innenraum ein bestimmtes elektrisches Feld ausbildet. Das Mikrosystem, insbesondere der Innenraum, ist mit der Trägerflüssigkeit gefüllt oder durchströmt. Befindet sich in der Trägerflüssigkeit ein suspendiertes Teilchen (zu halterndes oder zu führendes Objekt), so wird dieses unter Wirkung des elektrischen Feldes polarisiert. Werden die Elektroden mit Hochfrequenzsignalen (MHz-Bereich) angesteuert oder ist die Außenleitfähigkeit (der Trägerflüssigkeit) höher als die mittlere Innenleitfähigkeit des oder der Teilchen, so werden diese von den Elektroden weg in den Innenraum hin zu einem Feldminimum gedrückt. Im Bereich des Feldminimums werden die Teilchen freischwebend in der Trägerflüssigkeit gehalten (geschlossener Feldkäfig). Sind die Elektroden so angeordnet und gesteuert, daß sich ein Potentialwall nur in Bezug auf eine Raumrichtung ausbildet, eine Bewegung der Teilchen jedoch insbesondere unter der Wirkung eines Flüssigkeitsströmung in eine andere Raumrichtung möglich ist, so kann sich das Teilchen im Feldkäfig in eine bestimmte Richtung bewegen (offener Feldkäfig).

Die Grundpotentiale der Elektroden sind so gewählt, daß sich das Teilchen an einer vorbestimmten Position im Feldkäfig befindet. Diese vorbestimmte Position ist der Ort des Feldminimums beim geschlossenen Feldkäfig bzw. die Bahn (Trajektorie) im offenen Feldkäfig. Erfindungsgemäß wird durch periodische und geordnete Überlagerung der Grundpotentiale mit Steuerpotentialen (oder Änderung der Grundpotentiale gemäß den Steuerpotentialen) das tatsächliche Feldminimum von der vorbestimmten Position weg im Raum verschoben. Diese Verschiebung kann mit charakteristischen Zeiten um µs bis min erfolgen. Je nach der Trägheit der Objekte und der Viskosität der Lösung folgen die gehalterten Teilchen dem Feldminimum und bewegen sich somit auf vorgebbaren Bahnen in Bezug auf die gewünschte Position bzw. Bewegungsbahn.

Die überlagerte Bewegung kann je nach Anzahl der Elektroden und deren Ansteuerung eine einfache Vibration auf einer Raumachse oder eine komplexere Bahn im Raum sein. Die Erfindung erlaubt Bewegungen in allen Richtungen. Erfindungsgemäß ist es insbesondere möglich, zufällige oder unerwünschte Rotationsbewegungen des Teilchens durch Anlegen von Kompensationspotentialen auszugleichen. Die Kompensationspotentiale umfassen in geeigneter Weise rotierende oder sich bewegende elektrische Hochfrequenzfelder.

Die Ansteuerung der Elektroden erfolgt mit HF-Generatoren zur Bereitstellung der Grundpotentiale. Die Ausgangssignale der Generatoren werden periodisch oder aperiodisch (insbesondere in Reaktion auf ein Betätigungssignal, das eine bestimmte Objektposition anzeigt) amplitudengedämpft, phasenverschoben und/oder frequenzverändert. Diese jeweiligen Änderungen bestimmen die jeweiligen Steuerpotentiale, unter deren Wirkung die überlagerte Bewegung der gehalterten Teilchen erfolgt. Die Modulation der Steuerpotentiale kann in Bezug auf deren Amplitude, deren Phase oder deren Frequenz erfolgen. Es sind aber auch Kombinationen derartiger Modulationen möglich.

Die Elektrodenansteuerung erfolgt über ein schnelles Schaltmittel, insbesondere eine Multiplexeranordnung. Zur Realisierung komplexer Bewegungsmuster ist die Multiplexeranordnung vorzugsweise rechnergesteuert und nach vorgebbaren Programmen regelbar.

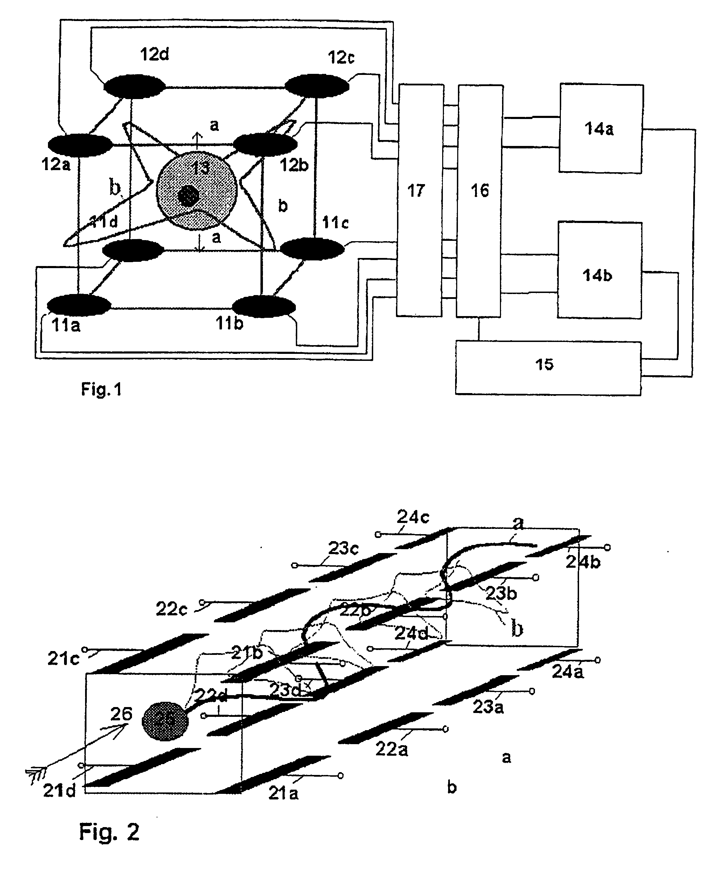

Fig. 1 zeigt als Beispiel eine Quadrupolanordnung, bei der acht Elektroden 11a-11d, 12a-12d in Form eines Kubus angeordnet sind. Durch Anlegen hochfrequenter Wechselspannungen an den Elektroden wird in bekannter Weise ein elektrisches Feld mit einem Feldminimum im Schwerpunkt des Kubus gebildet. Die Begrenzungen des Feldkäfigs sind schematisch durch durchgezogene Linien zwischen den Elektroden dargestellt. Im Innenraum der Elektrodenanordnung befindet sich eine Trägerflüssigkeit mit einem suspendierenden dielektrischen Partikel 13 (z.B. ein Latexteilchen von wenigen Mikrometer Durchmesser oder eine biologische Zelle). Das dielektrische Partikel wird so polarisiert, daß negative Influenzladungen zu der jeweils nächsten negativen Elektrode (und umgekehrt) zeigen. Dadurch wird auf dem Partikel ein Oktupol influenziert und das Partikel in das Feldminimum geschoben.

Im rechten Teil von Fig. 1 ist ein Blockschaltbild einer Ansteuerungschaltung für die Elektroden dargestellt. Die Steuerschaltung umfaßt mindestens einen HF-Generator, der mit einem als schnelles Schaltmittel wirkenden Multiplexerbaustein 16 verbunden ist. Im dargestellten Fall sind zwei HF-Generatoren 14a, 14b vorgesehen. Die Zahl der HF-Generatoren wird anwendungsabhängig danach ausgewählt, ob 2, 3, 4 oder mehr phasen- oder frequenzverschobene Signale zur Elektrodenansteuerung verwendet werden sollen. Zwischen dem Multiplexerbaustein 16 und den Elektroden ist ein Kalibrierglied 17 vorgesehen, das zur schnellen Änderung der Amplitude, der Frequenz und/oder der Phasenlage jedes Potentials mit dem Multiplexerbaustein zusammenwirkt. Der Multiplexerbaustein 16 und das Kalibrierglied 17 sind vorzugsweise mit einem Rechner steuerbar oder in vorbestimmter Weise voreinstellbar. Die Signale der HF-Generatoren 14a, 14b werden mit dem Multiplexerbaustein und dem Kalibrierglied 17 in der unten unter Bezug auf Tabelle 1 erläuterten Weise so aufgespalten, daß zwischen den Elektroden 11a-11d, 12a-12d ein Feldkäfig gebildet wird.

In Tabelle 1 ist die Ansteuerung der Elektroden des Oktupols mit Grundpotentialen einer bestimmten Amplitude (im Bereich von 0,1 V bis 100 V) und einer bestimmten Frequenz (MHz-Bereich) dargestellt. Zur Bildung eines geschlossenen Feldkäfigs unterscheiden sich die Grundpotentiale der einzelnen Elektroden durch die gegenseitige Phasenlage. Es wird ein zweiphasiges Wechselfeld und ein vierphasiges Rotationsfeld ausgebildet, wobei die relative Phasenlage im wesentlichen den in Tabelle 1 dargestellten Werten entspricht.

Das mit einer Elektrodenansteuerung gemäß Tabelle 1 erzeugte Feldminimum im Schwerpunkt des Feldkäfigs wird erfindungsgemäß so bewegt, daß ein sich im Feldminimum befindliches Teilchen z.B. den Bewegungsbahnen (a) oder (b) (s. Fig. 1) folgt. Die Bewegungsbahn (a) ist eine Vibration in einer Raumrichtung (zwei Bewegungsabläufe pro Periode). Die Bewegungsbahn (b) ist eine komplexe, sternförmige Bahn im Feldkäfig (acht Bewegungsabläufe pro Periode). Je nach Ansteuerung der Elektroden lassen sich auch beliebige andere Trajektorien realisieren.

Die genannten Bewegungen des Feldminimums werden erzielt, indem die Grundpotentiale gemäß Tabelle 1 mit Steuerpotentialen überlagert werden, deren Parameter beispielhaft in Tabelle 2 zusammengestellt sind.

Die oberen 6 Zeilen von Tabelle 2 beziehen sich auf die in Fig. 1 gezeigte Bewegungsart (a). Jeweils 2 Zeilen beziehen sich auf die beiden gegenläufigen Bewegungsabläufe 1, 2 der Vibration, wobei die drei Ansteuerungsmöglichkeiten der Beeinflussung der Amplituden, Frequenzen oder Phasenlagen angegeben sind. Die Phasen ϕ₁, ϕ₂ bei der dritten Möglichkeit (Zeilen 5, 6) sind beliebig unter Bildung eines festen Differenzwertes ausgewählt. Der Differenzwert beträgt vorzugsweise ϕ₁ - ϕ₂ = 180°, es sind jedoch auch andere Differenzwerte möglich. Entsprechendes gilt für die amplituden- und frequenzbezogene Ansteuerung. Die Zeilen 1 und 2 bzw. 3 und 4 zeigen hierzu jeweils Beispielwerte.

Die unteren 8 Zeilen von Tabelle 2 beziehen sich auf die zeitliche Abfolge der 8 Bewegungsabläufe zur Bildung der sternförmigen Bewegungsbahn (b) bei amplitudenbezogener Gestaltung der Steuerpotentiale. Wiederum sind hier die Amplitudenwerte nur beispielhaft angegeben.

Dem Fachmann ist erkennbar, daß sich die erfindungsgemäße Verfahrensweise nicht auf die in Tabelle 2 angegebenen Beispielwerte oder Gestaltungsarten der Steuerpotentiale beschränkt. Es sind vielmehr auch Mischformen der amplituden-, frequenz- und phasenbezogenen Gestaltungen der Steuerpotentiale möglich. In jedem Fall kommt es darauf an, daß zur Ausbildung einer überlagerten Bewegung in Bezug auf eine gewünschte Objektposition die Steuerpotentiale im Zeitablauf derart gewählt werden, daß ein Feldkäfig mit einem in einer Raumrichtung verminderten Potentialwall gebildet wird, wobei sich die Raumrichtung dieser Feldkäfigschwächung im Zeitablauf periodisch in Bezug auf die gewünschte Objektposition oder -bahn verändert.

Durch die Ansteuerung der Elektrodenanordnung mit Potentialen, die durch die Überlagerung der Grundpotentiale gemäß Tabelle 1 und Steuerpotentiale gemäß Tabelle 2 gebildet werden, wird das Feldminimum im Feldkäfig gemäß Fig. 1 bewegt. Das eingefangene Teilchen wird dieser Bewegung folgen, da es bestrebt ist, eine Position minimaler Energie einzunehmen.

Der in Fig. 1 gezeigte Steuerrechner 15 kann im einfachsten Fall durch einen Multivibrator ersetzt werden, der wechselweise die Amplituden der oberen Elektrodenebene 12 und der unteren Elektrodenebene 11 halbiert. In diesem Fall wird der Bewegungsablauf (a) erreicht.

Die Steuerpotentiale (Signalmodulationen gemäß Tabelle 2) bzw. die Perioden der überlagerten Bewegunen besitzen charakteristische Zeitkonstanten der Größenordnung Mikrosekunden bis Minuten. Die durch die Feldminimumverlagerung erzielten Bahndurchmesser bzw. Auslenkungen können im Bereich von 50 nm bis einige µm liegen. Abweichend von den Bahnformen (a) und (b) können kompliziertere Bahnformen bei entsprechender Gestaltung der Steuerpotentiale gebildet werden.

In Fig. 2 ist ein Beispiel eines offenen Feldkäfigs dargestellt, der aus einer linearen Folge mehrerer Quadrupole 21a-21d, 22a-22d, 23a-23d, 24a-24d gebildet wird. Die Elektrodenanordnung bildet einen Kanal, in dem mit einer Flüssigkeitsströmung 26 ein Teilchen 25 geführt wird. Die beispielhaft dargestellten Teilchenbahnen (a) und (b) lassen sich bei geeigneter Ansteuerung der Elektroden vorgeben. In Tabelle 3 ist die Elektrodenansteuerung für die dick durchgezogene Bewegungsbahn (a) angegeben. In einem ersten Schritt (erste Zeile) werden die Elektroden so angesteuert, daß der Feldkäfig im Bereich der Elektroden 21a-d eine Schwächung in die Richtung der Ebene besitzt, die durch die Elektroden 21a und 21b gebildet wird. Hierfür ist das Potential der letzteren Elektroden vermindert. Im zweiten Schritt erfolgt eine Bewegungsumkehr durch Schwächung des Feldkäfigs auf der entgegengesetzten Seite (Reduzierung der Potentiale an den Elektroden 22c, d). Der weitere Ablauf erfolgt entsprechend.

Vorteilhafterweise muß nicht bekannt sein, an welcher Stelle sich ein Partikel befindet. Allein durch die periodische Wiederholung der in Tabelle 3 beispielhaft angegebenen Signalfolgen kann die gewünschte Bewegung erzielt werden. Vorzugsweise erfolgt die Wiederholung mit einer Frequenz, deren Kehrwert etwa 1/10 der Zeit, die das Teilchen für das Durchlaufen einer Quadupolanordnung benötigt, oder größer ist.

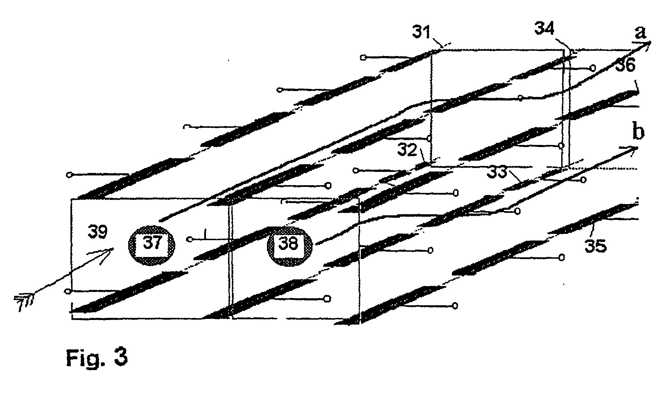

Eine Elektrodenanordnung gemäß Fig. 3 ist zum Ein- bzw. Auskoppeln von Teilchen aus einem Teilchenstrom eingerichtet. Die einzeln oder in Gruppen ansteuerbaren Elektroden 31-36 werden in mehreren Reihen angeordnet und sind von einer freien Suspension durchströmbar. Sie sind in beliebigen Richtungen, vorzugsweise parallel zu den Elektrodenreihen durchströmbar.

Mit Ansteuerprotokollen, die analog zu den oben erläuterten Beispielen gestaltet sind, lassen sich die Bewegungsbahnen (a) und (b) erzielen. Bei Anbringung gefächerter Anschlußmittel (Mehrkanalanschluß) können damit Teilchen separiert und sortiert werden.

**Tab. 1:**

| Phasenlage ϕ bei Ansteuerung der 8 Elektroden gemäß Figur 1 mit Grundpotentialen zur Bildung eines geschlossenen Feldkäfigs mit einem Feldminimum (Grundeinstellung) | | | | | | | | | |
|---|---|---|---|---|---|---|---|---|---|
| Grundansteuerung | Periode Grundsignal | E1. 11a | E1. 11b | E1. 11c | E1. 11d | E1. 12a | E1. 12b | E1. 12c | E1. 12d |
| Wechselfeld | 1 | 0° | 180° | 0° | 180° | 180° | 0° | 180° | 0° |
| | 2 | 180° | 0° | 180° | 0° | 0° | 180° | 0° | 180° |
| Rotat.-feld 4 Phasen | 1 | 0° | 90° | 180° | 270° | 180° | 270° | 0° | 90° |
| | 2 | 90° | 180° | 270° | 0° | 270° | 0° | 90° | 180° |
| | 3 | 180° | 270° | 0° | 90° | 0° | 90° | 180° | 270° |
| | 4 | 270° | 0° | 90° | 180° | 90° | 180° | 270° | 0° |

**Tab. 2:**

| Ansteuerung der Elektroden gemäß Figur 1 zur Erzeugung der Bewegungsbahnen (a) und (b) | | | | | | | | | |
|---|---|---|---|---|---|---|---|---|---|
| Bahn | Bew.-ablauf | E1. 11a | E1. 11b | E1. 11c | E1. 11d | E1. 12a | E1. 12b | E1. 12c | E1. 12d |
| a | 1 | Ampl. U | Ampl. U | Ampl. U | Ampl. U | Ampl. U/4 | Ampl. U/4 | Ampl. U/4 | Ampl. U/4 |
| | 2 | Ampl. U/4 | Ampl. U/4 | Ampl. U/4 | Ampl. U/4 | Ampl. U | Ampl. U | Ampl. U | Ampl. U |
| a | 1 | Frequ. f | Frequ. f | Frequ. f | Frequ. f | Frequ. f/10 | Frequ. f/10 | Frequ. f/10 | Frequ. f/10 |
| | 2 | Frequ. f/10 | Frequ. f/10 | Frequ. f/10 | Frequ. f/10 | Frequ. f | Frequ. f | Frequ. f | Frequ. f |
| a | 1 | Phase ϕ1 | Phase ϕ2 | Phase ϕ1 | Phase ϕ2 | Phase ϕ1 | Phase ϕ1 | Phase ϕ1 | Phase ϕ1 |
| | 2 | Phase ϕ2 | Phase ϕ2 | Phase ϕ2 | Phase ϕ2 | Phase ϕ1 | Phase ϕ2 | Phase ϕ1 | Phase ϕ2 |
| b | 1 | Ampl. U/4 | Ampl. U | Ampl. U | Ampl. U | Ampl. U/4 | Ampl. U | Ampl. U | Ampl. U |
| | 2 | Ampl.U | Ampl.U | Ampl.U | Ampl.U | Ampl.U | Ampl.U | Ampl.U | Ampl.U |
| | 3 | Ampl. U | Ampl. U/4 | Ampl. U | Ampl. U | Ampl. U | Ampl. U/4 | Ampl. U | Ampl. U |
| | 4 | Ampl.U | Ampl.U | Ampl.U | Ampl.U | Ampl.U | Ampl.U | Ampl.U | Ampl.U |
| | 5 | Ampl. U | Ampl. U | Ampl. U/4 | Ampl. U | Ampl. U | Ampl. U | Ampl. U/4 | Ampl. U |
| | 6 | Ampl.U | Ampl.U | Ampl.U | Ampl.U | Ampl.U | Ampl.U | Ampl.U | Ampl.U |
| | 7 | Ampl. U | Ampl. U | Ampl. U | Ampl. U/4 | Ampl. U | Ampl. U | Ampl. U | Ampl. U/4 |
| | 8 | Ampl.U | Ampl.U | Ampl.U | Ampl.U | Ampl.U | Ampl.U | Ampl.U | Ampl.U |

**Tab. 3:**

| Ansteuerung der Elektroden gemäß Figur 2 zur Erzeugung der Bewegungsbahn (a) | | | | | | | | | | | | | | | | | |
|---|---|---|---|---|---|---|---|---|---|---|---|---|---|---|---|---|---|
| | | Elektrode 21 | | | | Elektrode 22 | | | | Elektrode 23 | | | | Elektrode 24 | | | |
| Bahn Bew abl | | a | b | c | d | a | b | c | d | a | b | c | d | a | b | c | d |
| a | 1 | U/4 | U/4 | U | U | U | U | U | U | U | U | U | U | U | U | U | U |
| | 2 | U | U | U | U | U | U | U/4 | U/4 | U | U | U | U | U | U | U | U |
| | 3 | U | U | U | U | U | U | U | U | U/4 | U/4 | U | U | U | U | U | U |
| | 4 | U | U | U | U | U | U | U | U | U | U | U | U | U | U | U/4 | U/4 |

## Patentansprüche

1. Verfahren zur Positionssteuerung mindestens eines Objektes (13, 25, 37, 38) an vorbestimmten Positionen in einer Elektrodenanordnung mit einer Vielzahl von Elektroden (11a-11d, 12a-12d, 21a-21d, 22a-22d, 23a-23d, 24a-24d, 31-36), die mit elektrischen hochfrequenten Grundpotentialen beaufschlagt werden, um auf das Objekt elektrische Polarisationskräfte auszuüben und einen Feldkäfig mit mindestens einem Feldminimum zu bilden,
**dadurch gekennzeichnet, daß**
mindestens eines der hochfrequenten Grundpotentiale derart mit mindestens einem Steuerpotential moduliert wird, daß sich die Position des Feldminimums, in dem sich das Objekt im Feldkäfig befindet, in Bezug auf die vorbestimmte Position andert.

2. Verfahren gemäß Anspruch 1, bei der mit den Grundpotentialen ein geschlossener Feldkäfig gebildet wird und die Änderung der Position des Feldminimums zeitlich und räumlich periodisch ist.

3. Verfahren gemäß Anspruch 2, bei dem das Steuerpotential durch Modulationen der Amplituden, der Phasenlage und/oder der Frequenzen der Grundpotentiale gebildet wird.

4. Verfahren gemäß Anspruch 3, bei dem das Feldminimum mit dem Objekt einen geschlossenen Bahnverlauf bildet.

5. Verfahren gemäß einem der Ansprüche 1-4, bei dem die zeitliche Änderung der Position des Feldminimums mit einer charakteristischen Zeit erfolgt, die wesentlich länger als der Kehrwert der Frequenz des Grundpotentials ist.

6. Verfahren gemäß Anspruch 1, bei dem das Grundpotential zur Bildung eines offenen Feldkäfigs eingerichtet ist und die Steuerpotentiale so ausgewählt werden, daß sich das Objekt in Bezug auf eine vorbestimmte Bahn bewegt.

7. Verfahren gemäß einem der Ansprüche 1-5 oder 6, bei dem die Steuerpotentiale Zeitmuster zur periodischen Änderung der Position des Feldminimums durchlaufen.

8. Verfahren gemäß einem der vorhergehenden Ansprüche, bei dem eine Vielzahl von Objekten in einer Elektrodenanordnung positioniert werden.

9. Vorrichtung zur Anordnung mindestens eines Objekts (13, 25, 37, 38) an vorbestimmten Positionen in einer Elektrodenanordnung mit einer Vielzahl von Elektroden (11a-11d, 12a-12d, 21a-21d, 22a-22d, 23a-23d, 24a-24d, 31-36) zur Bildung eines Feldkäfigs mit mindestens einem Feldminimum unter Wirkung elektrischer Polarisationskräfte, wobei die Elektroden mit Generatormitteln mit elektrischen hochfrequenten Grundpotentialen beaufschlagbar sind,
**gekennzeichnet durch**
Schaltmittel zur Modulation der hochfrequenten Grundpotentiale entsprechend vorbestimmten Steuerpotentialen.

10. Vorrichtung gemäß Anspruch 9, bei der die Schaltmittel durch einen Multiplexer zur selektiven Ansteuerung der Elektroden gebildet werden.

## Claims

1. Method for position control of at least one object (13, 25, 37, 38) at predetermined positions in an electrode arrangement with a multiplicity of electrodes (11a - 11d, 12a - 12d, 21a - 21d, 22a - 22d, 23a - 23d, 24a - 24d, 31 - 36), which can be supplied with electrical high frequency basic potentials in order to exert electrical polarisation forces on the object and to form a field cage with at least one field minimum,
**characterised in that**
at least one of the high frequency basic potentials is modulated with at least one control potential in such a manner that the position of the field minimum, in which the object is situated in the field cage, changes with respect to the predetermined position.

2. Method according to claim 1, in which a closed field cage is formed with the basic potentials and the change of the position of the field minimum is temporally and spatially periodic.

3. Method according to claim 2, in which the control potential is formed by means of modulations of the amplitudes, of the phase position and/or of the frequencies of the basic potentials.

4. Method according to claim 3, in which the field minimum forms a closed track configuration with the object.

5. Method according to one of the claims 1 - 4, in which the temporal change of the position of the field minimum is effected with a characteristic time which is substantially longer than the reciprocal value of the frequency of the basic potential.

6. Method according to claim 1, in which the basic potential is set in order to form an open field cage and the control potentials are chosen such that the object moves with respect to a predetermined track.

7. Method according to one of the claims 1 - 5 or 6, in which the control potentials pass through time patterns for periodic alteration of the position of the field minimum.

8. Method according to one of the preceding claims, in which a multiplicity of objects is positioned in an electrode arrangement.

9. Device for disposing at least one object (13, 25, 37, 38) at predetermined positions in an electrode arrangement with a multiplicity of electrodes (11a - 11d, 12a - 12d, 21a - 21d, 22a - 22d, 23a - 23d, 24a - 24d, 31 - 36) for forming a field cage with at least one field minimum under the effect of electrical polarisation forces, the electrodes being able to be supplied by generator means with electrical high frequency basic potentials,
**characterised by**
switching means for modulation of the high frequency basic potentials corresponding to predetermined control potentials.

10. Device according to claim 9, in which the switching means are formed by a multiplexor for selective actuation of the electrodes.

## Revendications

1. Procédé de commande de positionnement d'au moins un objet (13, 25, 37, 38) à des positions prédéterminées dans un agencement d'électrodes avec une pluralité d'électrodes (11a-11d 12a-12d, 21a-21d, 22a-22d, 23a-23d, 24a-24d, 31-36) qui sont alimentées avec des potentiels électriques de base haute fréquence afin d'exercer des forces de polarisation électriques sur l'objet et former un piège magnétique avec au moins un minimum de champ, **caractérisé en ce qu'**au moins l'un des potentiels de base haute fréquence est modulé par au moins un potentiel de commande de telle sorte que la position du minimum de champ dans lequel se trouve l'objet dans le piège magnétique, change par rapport à la position prédéterminée.

2. Procédé selon la revendication 1, dans lequel un piège magnétique fermé est formé par les potentiels de base et le changement de la position du minimum de champ est périodique dans le temps et dans l'espace.

3. Procédé selon la revendication 2, dans lequel le potentiel de commande est formé par des modulations des amplitudes, des relations des phases et/ou des fréquences des potentiels de base.

4. Procédé selon la revendication 3, dans lequel le minimum de champ forme avec l'objet une trajectoire d'orbite fermée.

5. Procédé selon l'une quelconque des revendications 1 à 4, dans lequel le changement temporel de la position du minimum de champ a lieu avec un temps caractéristique qui est nettement plus long que la valeur réciproque de la fréquence du potentiel de base.

6. Procédé selon la revendication 1, dans lequel le potentiel de base est disposé pour former un piège magnétique ouvert et les potentiels de commande sont sélectionnés de telle sorte que l'objet se déplace par rapport à une orbite prédéterminée.

7. Procédé selon l'une quelconque des revendications 1 à 5 ou 6, dans lequel les potentiels de commande passent par des motifs temporels pour un changement périodique de la position du minimum de champ.

8. Procédé selon l'une quelconque des revendications précédentes, dans lequel une pluralité d'objets est positionnée dans un agencement d'électrodes.

9. Dispositif pour agencer au moins un objet (13, 25, 37, 38) à des positions prédéterminées dans un agencement d'électrodes avec une pluralité d'électrodes (11a-11d, 12a-12d, 21a-21d, 22a-22d, 23a-23d, 24a-24d, 31-36) pour former un piège magnétique avec au moins un minimum de champ sous l'influence de forces de polarisation électriques, les électrodes pouvant être alimentées par des moyens de générateur avec des potentiels électriques de base haute fréquence, **caractérisé par** des moyens de commutation pour moduler les potentiels de base haute fréquence selon des potentiels de commande prédéterminés.

10. Dispositif selon la revendication 9, dans lequel les moyens de commutation sont constitués par un multiplexeur pour l'excitation sélective des électrodes.
